# EUROPÄISCHE PATENTSCHRIFT

(11) **EP 1 532 447 B1**
(45) Veröffentlichungstag und Bekanntmachung des Hinweises auf die Patenterteilung: **27.02.2008**
(21) Anmeldenummer: 03794917.9
(22) Anmeldetag: 21.08.2003
(51) Int. Cl.: G01N 33/533

(54) **ERZEUGUNG VON CHEMILUMINESZENZ DURCH WASSERSTOFF**
GENERATION OF CHEMILUMINESCENCE BY HYDROGEN
GENERATION DE CHIMILUMINESCENCE A L'AIDE D'HYDROGENE

(30) Priorität: 26.08.2002 DE 10239098
(43) Veröffentlichungstag der Anmeldung: 25.05.2005
(73) Patentinhaber: F. HOFFMANN-LA ROCHE AG, 4070 Basel (CH); Roche Diagnostics GmbH, 68305 Mannheim (DE)
(72) Erfinder: GIESEN, Ursula, 82362 Weilheim (DE); HEINZE, Jürgen, 79104 Freiburg (DE); BORGWARTH, Kai, 79100 Freiburg (DE)
(74) Vertreter: Weiss, Wolfgang
(86) Internationale Anmeldenummer: PCT/EP2003/009299
(87) Internationale Veröffentlichungsnummer: WO 2004/025299

(56) Entgegenhaltungen:
- WO-A-02/00726
- CA-A- 2 313 144
- US-B1- 6 271 041
- US-B1- 6 432 722
- YANG MINLI ET AL: "Electrochemiluminescence assay for the detection of acridinium esters" ANALYTICA CHIMICA ACTA, Bd. 461, Nr. 1, 12. Juni 2002 (2002-06-12), Seiten 141-146, XP002262847 ISSN: 0003-2670

## Beschreibung

Die vorliegende Erfindung betrifft ein Verfahren zur Erzeugung von Chemilumineszenz umfassend die Bereitstellung einer chemilumineszierenden Spezies durch naszierenden Wasserstoff. Insbesondere betrifft die Erfindung ein Verfahren zum Nachweis eines Analyten in einer Probe unter Verwendung eines lumineszierenden Metallkomplexes als Markierungsgruppe und eine dafür geeignete Vorrichtung.

Lumineszierende Metallkomplexe sind aus dem Stand der Technik bekannt. EP-A-0 178 450 offenbart Rutheniumkomplexe, die an ein immunologisch aktives Material gekoppelt sind, wobei die Rutheniumkomplexe drei gleiche oder verschiedene bi- oder polycyclische Liganden mit mindestens zwei stickstoffhaltigen Heterocyclen enthalten, wobei mindestens einer dieser Liganden mit mindestens einer wasserlöslich machenden Gruppe, wie SO₃H oder -COOH, substituiert ist und wobei mindestens einer dieser Liganden mit mindestens einer reaktiven Gruppe wie -COOH direkt oder über eine Spacergruppe substituiert ist und wobei die Liganden über Stickstoffatome an das Ruthenium gebunden sind.

Weiterhin ist die Verwendung von lumineszierenden Metallkomplexen als Markierungsreagenzien für ein Elektrochemilumineszenz-Nachweisverfahren bekannt (vgl. z.B. EP-A-0 580 979, WO 87/06706, US 5,238,108 oder US 5,310,687). Ein solches Elektrochemilumineszenz-Nachweisverfahren beruht darauf, dass in einer geeigneten Messvorrichtung das Zentralatom des Metallkomplexes, z.B. Ruthenium, durch Elektronentransfer in den angeregten MLCT-Triplettzustand überführt wird. Von diesem angeregten Zustand kann es unter Emission eines Photons durch einen verbotenen Triplett-Singulett-Übergang in den Grundzustand relaxieren (vgl. z.B. WO/90 05296, Leland und Powell, J. Electrochem. Soc. 137 (1990), 3127-3131; Blackburn et al., Clin. Chem. 37 (1991), 1534-1539).

Der in der Literatur beschriebene Reaktionsmechanismus für die Erzeugung von Chemilumineszenz umfasst die Oxidation eines Mediators, wie Tripropylamin, zu einem Radikalkation. Dieses Radikalkation wird unter Protonenverlust zu einem TPA-Radikal. Das TPA-Radikal ist wiederum dasjenige Molekül, welches durch einen weiteren Elektronenübergang einen oxidierten Metallkomplex, z.B. einen Ru³⁺-Komplex, in den Ru²⁺-MLCT-Triplettzustand überführt, welcher in der Lage ist, ein Photon zu emittieren.

Mit dem geschilderten Mechanismus lassen sich jedoch einige experimentelle Befunde nicht erklären. So werden nur 40-50 % des theoretischen Stromes gefunden. Weiterhin ist die Erzeugung von Elektrochemilumineszenz in hohem Maße vom Elektrodenmaterial abhängig, was anhand der Funktion der Elektrode als Oxidationsmittel von TPA und dem Metallkomplex eigentlich nicht der Fall sein dürfte. Weiterhin wurden bisher auch keine TPA-Dimere nachgewiesen, die - falls TPA-Radikale gemäß obigem, Mechanismus entstehen - in Lösung gebildet werden sollten.

US 6,432,722 beschreibt ein Verfahren zur Detektion eines Analyten in einer Probe durch Elektrochemilumineszenzmessung. Dabei wird ein Metallkomplex über einen Mediator, bei dem es sich vorzugsweise um ein tertiäres Amin handelt, in einen lumineszenzfähigen Zustand angeregt. US6,271,041 beschreibt ebenfalls die Anregung der Chemilumineszenz eines Metallkomplexes über ein Amin. In beiden Dokumenten ist die Verwendung eines Mediators ein wesentliches Element für die Bildung einer chemilumineszenzfähigen Spezies. Andere Möglichkeiten zur Erzeugung einer chemilumineszenzfähigen Form eines Metallkomplexes sind nicht offenbart.

Es wurden daher weitere Untersuchungen zur Erzeugung von Chemilumineszenz bei Metallkomplexen durchgeführt. Dabei wurde überraschenderweise gefunden, dass ein Rutheniumkomplex in Gegenwart von naszierendem Wasserstoff, z.B. erzeugt durch Lithium/Butanol/H₂SO₄, Chemilumineszenz in hoher Ausbeute zeigt.

Auf Basis dieser neuen Erkenntnisse kann ein neues Verfahren zur Erzeugung von Chemilumineszenz mit einem Metallkomplex als Lumineszenzgenerator bereitgestellt werden, welches die Verwendung von naszierendem Wasserstoff zur Reduktion von oxidierten Metallkomplexen in den angeregten chemilumineszenzfähigen Zustand umfasst. Dieses Verfahren kann insbesondere zum Nachweis von Analyten in einer Probe eingesetzt werden, wobei gegenüber bisher verwendeten Verfahren eine verbesserte Chemilumineszenzausbeute oder/und eine verringerte Störanfälligkeit erzielt werden kann.

Ein erster Aspekt der Erfindung ist somit ein Verfahren zur Erzeugung von Chemilumineszenz mit einem lumineszierenden Metallkomplex als Lumineszenzgenerator, umfassend das Oxidieren des Metallkomplexes und das Reduzieren des Metallkomplexes durch naszierenden Wasserstoff, wobei eine chemilumineszenzfähige Form des Metallkomplexes entsteht.

Insbesondere betrifft die Erfindung ein Verfahren zum Nachweis eines Analyten in einer Probe unter Verwendung eines lumineszierenden Metallkomplexes als Markierungsgruppe, wobei die Lumineszenz des Metallkomplexes erzeugt wird durch die Schritte:
(i) Oxidieren des Metallkomplexes und
(ii) Reduzieren des Metallkomplexes durch naszierenden Wasserstoff, wobei eine chemilumineszenzfähige Form des Metallkomplexes entsteht, und
(iii) Bestimmen des Analyten über die Chemilumineszenz.

Ein weiterer Aspekt der Erfindung ist eine Vorrichtung zur Erzeugung von Chemilumineszenz unter Verwendung eines lumineszierenden Metallkomplexes als Lumineszenzgenerator, umfassend:
(i) Mittel zum Oxidieren des Metallkomplexes und
(ii) Mittel zum Erzeugen von naszierendem Wasserstoff,
wobei die Mittel (i) und (ii) zwei separate Reaktionskammern umfassen.

Insbesondere ist diese Vorrichtung vorgesehen zum Nachweis eines Analyten in einer Probe unter Verwendung eines lumineszierenden Metallkomplexes als Markierungsgruppe, umfassend:
(i) Mittel zum Oxidieren des Metallkomplexes,
(ii) Mittel zum Erzeugen von naszierendem Wasserstoff und
(iii) Mittel zum Nachweis von Lumineszenz,
wobei die Mittel (i) und (ii) zwei separate Reaktionskammern umfassen.

Besonders bevorzugt dient das Verfahren für Anwendungen auf dem Gebiet der Diagnostik, d.h. zum Nachweis eines Analyten in einer Probe. Beispielsweise kann das Verfahren zum Nachweis von physikalischen, chemischen oder biochemischen Parametern in einer Probe, z.B. einer Körperflüssigkeit, einer Gewebeprobe etc. oder einer Umweltprobe, eingesetzt werden.

Der Nachweis eines Analyten umfasst das Inkontaktbringen einer Probe mit einem Nachweisreagenz, das als Markierungsgruppe einen lumineszierenden Metallkomplex trägt. Die Probe ist vorzugsweise eine biologische Probe und liegt in flüssiger Form vor. Sie kann aus menschlichen, tierischen oder pflanzlichen Geweben, Körperflüssigkeiten, prokaryontischen oder eukaryontischen Zellkulturen etc. stammen.

Das Nachweisreagenz umfasst einen lumineszierenden Metallkomplex als Markierungsgruppe, der vorzugsweise an eine biologische Substanz, z.B. Biotin, Nukleinsäuren, z. B. Oligonukleotide, DNA oder RNA, Nukleinsäureanaloga, wie etwa peptidische Nukleinsäuren, Antikörper oder Antikörperfragmente, Peptid- oder Polypeptidantigene, d.h. immunologisch reaktive Polypeptide oder Haptene, d.h. organische Moleküle mit einem Molekulargewicht von 150 bis 2000, gekoppelt ist, sowie gegebenenfalls weitere Reagenzien, wie sie dem Fachmann bekannt sind.

Die Durchführung des erfindungsgemäßen Nachweisverfahrens umfasst vorzugsweise eine Inkubation der Probe mit dem Nachweisreagenz, um eine direkte oder indirekte Reaktion des Nachweisreagenz mit in der Probe vorhandenem Analyten zu bewirken. Das Vorhandensein eines Analyten bzw. dessen Menge in der Probe wird auf Basis des von der Markierungsgruppe stammenden Chemilumineszenzsignals qualitativ oder/und quantitativ ermittelt.

Das Verfahren kann als homogener Assay, d.h. Messung der Chemilumineszenz in einer Flüssigphase, durchgeführt werden. Vorzugsweise wird jedoch ein heterogener Test durchgeführt, bei dem die Chemilumineszenz-Markierung an einer Festphase, z.B. einer partikulären Festphase, wie etwa magnetischen Mikrobeads, z.B. Streptavidinbeschichteten Mikrobeads, oder kolloidalen Partikeln immobilisiert wird. Bei Durchführung eines heterogenen Tests kann das erfindungsgemäße Verfahren sogenannte Einfang- und Waschschritte umfassen, bei denen die Markierung auf der Festphase immobilisiert wird und eine Abtrennung der übrigen Probenbestandteile erfolgt.

Ein bevorzugte Merkmal des erfindungsgemäßen Verfahrens ist die Verwendung von chemilumineszenten Metallkomplexen, die eine Struktur der allgemeinen Formel (I) enthalten:

[M(L₁L₂L₃)]ₙ-Yₘ- (I)

worin
M ein zwei- oder dreiwertiges Metallkation ausgewählt aus Seltenerde- oder Übergangsmetallkationen ist,
L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
Y einen an einen der Liganden gebundenen Linker bedeutet, über den der Komplex, z.B. (a) an eine biologische Substanz gekoppelt ist oder (b) an eine biologische Substanz gekoppelt werden kann,
m eine ganze Zahl von 1 bis 10, vorzugsweise von 1 bis 4 und besonders bevorzugt von 1 ist und
n eine ganze Zahl von 1 bis 6, vorzugsweise 1 bis 3 und besonders bevorzugt von 1 ist.

Das Metallkation in diesem Komplex ist vorzugsweise Ruthenium, Osmium, Rhenium, Iridium, Rhodium, Platin, indium, Palladium, Molybdän, Techneticum, Kupfer, Chrom, Wolfram, Yttrium oder Lutetium. Besonders bevorzugt sind Ruthenium, Iridium, Rhenium, Chrom und Osmium. Am meisten bevorzugt ist Ruthenium. Zum Ladungsausgleich kann der Komplex gegebenenfalls noch Gegenionen, z.B. Anionen, enthalten.

Die Liganden L₁, L₂ und L₃ sind vorzugsweise Liganden mit mindestens 2 stickstoffhaltigen Heterocyclen. Bevorzugt sind aromatische Heterocyclen, wie z.B. Bipyridyl, Bipyrazyl, Terpyridyl und Phenanthronyl. Besonders bevorzugt werden die Liganden aus Bipyridin- und Phenanthrolin-Ringsystemen ausgewählt.

In den erfindungsgemäßen Metallkomplexen sind vorzugsweise hydrophile Gruppen oder/und Ladungsträger vorhanden, die beispielsweise kovalent gebunden, z.B. an den Linker oder an einen anderen Substitutenten der Liganden L₁, L₂ oder L₃, sind. Derartige hydrophile oder geladene Metallkomplexe sind z.B. aus WO 96/03409 und WO 96/03410 bekannt. Der Begriff "Ladungsträger" bedeutet im Sinne der vorliegenden Erfindung eine Gruppe, die bei einem pH-Wert in einem Bereich von 6 bis 8 überwiegend in ionischer Form vorliegt. Der Komplex enthält vorzugsweise bis zu 10, besonders bevorzugt 2 bis 8 solcher Ladungsträger.

Besonders bevorzugt enthält der Komplex mindestens einen negativen Ladungsträger. Beispiele für geeignete negative Ladungsträger sind Phosphat-, Phosphonat-, Sulfonat- und Carboxylatgruppen, wobei Sulfonat- und Carboxylatgruppen am meisten bevorzugt sind.

Weiterhin sind für das erfindungsgemäße Verfahren Komplexe geeignet, die eine hydrophile Gruppe enthalten. Beispiele für geeignete hydrophile Gruppen sind C₂-C₃-Alkylenoxy-Einheiten. C₂-C₃-Alkylenthio-Einheiten und Polyhydroxyeinheiten.

Die Herstellung solcher Metallkomplexe kann nach bekannten Methoden erfolgen, beispielsweise durch Reaktion eines Metallsalzes, z.B. eines Metallhalogenids, und gegebenenfalls anschließendem Austausch des Halogenidions durch Hexafluorophosphat-, Trifluoracetat- oder Tetrafluoroborat-Gruppen. Derartige Verfahren sind bekannt. Der Metallkomplex wird für das erfindungsgemäße Verfahren üblicherweise in Form von Konjugaten mit einer biologischen Substanz eingesetzt, wobei an die biologische Substanz mindestens ein Metallkomplex gekoppelt ist. Beispiele für geeignete biologische Substanzen sind Zellen, Viren, subzelluläre Teilchen, Proteine, Lipoproteine, Glycoproteine, Peptide, Polypeptide, Nukleinsäuren, Oligosaccharide, Polysaccharide, Lipopolysaccharide, zelluläre Metaboliten, Haptene, Hormone, pharmakologische Wirkstoffe, Alkaloide, Steroide, Vitamine, Aminosäuren und Zucker.

Die Kopplung des Metallkomplexes mit der biologischen Substanz erfolgt vorzugsweise über eine reaktive oder aktivierbare funktionelle Gruppe am Metallkomplex, z.B. ein Carbonsäurehalogenid, ein Carbonsäureanhydrid oder einen Aktivester, wie etwa ein N-Hydroxy-Succinimidester, oder ein Maleimid, die mit einer funktionellen Gruppe der biologischen Substanz kovalent koppeln kann. Wenn die funktionelle Gruppe ein Carbonsäureanhydrid, Carbonsäurehalogenid oder Aktivester ist, kann beispielsweise eine Kopplung mit freien Aminogruppen der biologischen Substanz erfolgen. Wenn die funktionelle Gruppe ein Maleimidrest ist, kann eine Kopplung mit freien SH-Gruppen der biologischen Substanz erfolgen. Auf analoge Weise kann auch eine Aktivierung von funktionellen Gruppen der biologischen Substanz erfolgen, die anschließend beispielsweise mit einer freien Carbonsäure-, Amino- oder Thiolfunktion des Metallkomplexes reagieren können.

Das erfindungsgemäße Verfahren umfasst die Schritte (i) Oxidieren des Metallkomplexes und (ii) Reduzieren des Metallkomplexes. Die Oxidation des Zentralatoms des Metallkomplexes kann elektrochemisch oder chemisch erfolgen. Für die elektrochemische Oxidation wird ein für das jeweilige Metallion ausreichendes anodisches Potential an eine Elektrode angelegt. Für den Übergang Ru²⁺/Ru³⁺ beträgt dieses Potential vorzugsweise mindestens + 1,2 V, besonders bevorzugt + 1,2 bis + 1,4 V (bezogen auf eine Ag/AgCI-Referenzelektrode). Alternativ kann die Oxidation des Zentralatoms des Metallkomplexes auch chemisch erfolgen. Beispiele für geeignete chemische Oxidationsmittel sind PbO₂, Permanganat, Cer⁴⁺-Verbindungen oder/und Peroxodisulfate.

Bei einer vorhergehenden chemischen Oxidation findet die nachfolgende Reduktion vorzugsweise räumlich oder/und zeitlich getrennt statt, beispielsweise in zwei separaten Reaktionskammern, wobei in der ersten Reaktionskammer die Oxidation und in der zweiten Kammer die Reduktion durchgeführt wird. Vor der Reduktion wird vorzugsweise überschüssiges Oxidationsmittel entfernt, z.B. durch Wegführen oder/und - im Falle eines heterogenen Tests mit einer Festphasen-gebundenen Markierungsgruppe - durch Waschen der Festphase. Alternativ kann ein Überschuss des Oxidationsmittels auch von einer dritten Substanz zerstört werden.

Wird eine elektrochemische Oxidation des Metallkomplexes durchgeführt, kann das Verfahren in einer einzigen Kammer durchgeführt werden, in die während des Reduktionsschrittes naszierender Wasserstoff erzeugt oder/und eingeleitet wird.

Der Reduktionsschritt des erfindungsgemäßen Verfahrens umfasst das Erzeugen von naszierendem Wasserstoff, um den oxidierten Metallkomlex in einen Zustand überzuführen, der die Emission eines Chemilumineszenzphotons erlaubt. Um eine möglichst hohe Effizienz der Reduktion zu erreichen, ist es bevorzugt, dass der naszierende Wasserstoff in unmittelbarer Nachbarschaft des Metallkomplexes, insbesondere in einem Abstand bis maximal 50 nm entsteht. Der naszierende Wasserstoff kann elektrochemisch, chemisch oder/und durch Ultraschall erzeugt werden. Die elektrochemische Erzeugung des naszierenden Wasserstoffs erfolgt vorzugsweise durch Anlegen einer Spannung von ≤ -1,0 V (bezogen auf eine Ag/AgCl-Referenzelektrode). Die chemische Erzeugung von naszierendem Wasserstoff kann durch bekannte Reagenzien, wie etwa Li/Butanol/H₂SO₄, Zn-Cu/Ethanol oder Zn/HCl, erfolgen. Die Erzeugung des naszierenden Wasserstoffs mittels Ultraschall erfolgt vorzugsweise durch Ablösen bzw. Herausschlagen von Wasserstoff-Radikalen aus organischen Verbindungen, insbesondere Alkylverbindungen. Die Ultraschallenergie liegt hierbei vorzugsweise im Bereich von 0,1-10 MHz, besonders bevorzugt bei ca. 1 MHz (Suslick & Price, Annu. Rev. Mater. Sci 29 (1999), 295; Mizik & Ries, Ann. NY Acad. Sci 899 (2000), 335).

Eine besonders bevorzugte Ausführungsform des erfindungsgemäßen Verfahren umfasst zunächst eine chemische Oxidation des Metallkomplexes und anschließend eine elektrochemische Erzeugung von naszierendem Wasserstoff, z.B. in einer elektrochemischen Zelle, welche den naszierenden Wasserstoff in hoher Konzentration zur Verfügung stellt. Beispiele für geeignete elektrochemische Zellen sind in EP-A-0 658 760 beschrieben. Auch bei dieser Ausführungsform ist es zweckmäßig, dass Oxidation und Erzeugung von naszierendem Wasserstoff in zwei getrennten Reaktionskammern erfolgen. Weiterhin soll die vorliegende Erfindung durch das nachfolgende Beispiel erläutert werden:

### Beispiel Chemilumineszenz durch naszierenden Wasserstoff

Ein Ruthenium(bipyridyl)₃-Komplex (mit einem Ru²⁺-Kation) wurde zu einem Ru³⁺-Komplex oxidiert. Hierzu wurde ein homogenes System aus Li/Butanol/H₂SO₄ genutzt. In ein Gefäß PbO₂ (Pulver) wurde konzentrierte Schwefelsäure gegeben. Hierüber wurde Butanol überschichtet. In dem Butanol war der Ru²⁺-Komplex in einer Konzentration von 1 mmol gelöst. An der Grenzfläche zwischen H₂SO₄ und Butanol findet die Oxidation des Ru²⁺-Komlexes statt. Nach erfolgter Oxidation des Ru²⁺-Komplexes zu Ru³⁺ wurde Lithium zugegeben. An der Grenzfläche zwischen H₂SO₄ und Butanol wird dabei naszierender Wasserstoff gebildet. An dieser Grenzfläche wurde eine ausgeprägte Ruthenium-Chemilumineszenz beobachtet.

Auch bei elektrochemischer Oxidation von Ru³⁺ aus Ru²⁺ und anschließender Erzeugung von naszierendem Wasserstoff durch Li/Butanol/H₂SO₄ konnte Ru-Chemitumineszenz beobachtet werden.

## Patentansprüche

1. Verfahren zum Nachweis eines Analyten in einer Probe unter Verwendung eines lumineszierenden Metallkomplexes als Markierungsgruppe,
**dadurch gekennzeichnet,**
**dass** eine Chemilumineszenz des Metallkomplexes angeregt wird durch die Schritte
(i) Oxidieren des Metallkomplexes und
(ii) Reduzieren des Metallkomplexes durch naszierenden Wasserstoff, wobei eine chemilumineszenzfähige Form des Metallkomplexes entsteht und
(iii) Bestimmen des Analyten über die Chemilumineszenz.

2. Verfahren nach Anspruch 1,
**dadurch gekennzeichnet,**
**dass** man einen Metallkomplex als Markierungsgruppe verwendet, der eine Struktur der allgemeinen Formel (I) enthält:
[M(L₁L₂L₃)]ₙ-Y-ₘ (I)
worin
M ein zwei- oder dreiwertiges Metallkation ausgewählt aus Seltenerde- oder Übergangsmetallkationen ist,
L₁, L₂ und L₃ gleich oder verschieden sind und Liganden mit mindestens zwei stickstoffhaltigen Heterocyclen bedeuten, wobei L₁, L₂ und L₃ über Stickstoffatome an das Metallkation gebunden sind,
Y einen an einen der Liganden gebundenen Linker bedeutet,
m eine ganze Zahl von 1 bis 10 ist und
n eine ganze Zahl von 1 bis 6 ist.

3. Verfahren nach Anspruch 1 oder 2,
**dadurch gekennzeichnet,**
**dass** man als Metallkomplex einen Rutheniumkomplex verwendet.

4. Verfahren nach einem der Ansprüche 1 bis 3,
**dadurch gekennzeichnet,**
**dass** die Liganden des Metallkomplexes ausgewählt werden aus Bipyridin- oder Phenanthrolin-Ringsystemen.

5. Verfahren nach einem der Ansprüche 1 bis 4,
**dadurch gekennzeichnet,**
**dass** der Metallkomplex mindestens eine hydrophile Gruppe oder/und einen Ladungsträger enthält.

6. Verfahren nach einem der Ansprüche 1 bis 5,
**dadurch gekennzeichnet,**
**dass** der Metallkomplex als Konjugat mit einem Nachweisreagenz für den Analyten verwendet wird.

7. Verfahren nach einem der Ansprüche 1 bis 6,
**dadurch gekennzeichnet,**
**dass** der Nachweis als homogener Test durchgeführt wird.

8. Verfahren nach einem der Ansprüche 1 bis 6,
**dadurch gekennzeichnet,**
**dass** der Nachweis als heterogener Test durchgeführt wird.

9. Verfahren nach einem der Ansprüche 1 bis 8,
**dadurch gekennzeichnet,**
**dass** die Oxidation des Metallkomplexes elektrochemisch erfolgt.

10. Verfahren nach Anspruch 9,
**dadurch gekennzeichnet,**
**dass** die Oxidation durch Anlegen eines anodischen Potentials von mindestens + 1,2 V (bezogen auf eine Ag/AgCl-Referenzelektrode) erfolgt.

11. Verfahren nach einem der Ansprüche 1 bis 8,
**dadurch gekennzeichnet,**
**dass** die Oxidation des Metallkomplexes chemisch erfolgt.

12. Verfahren nach Anspruch 11,
**dadurch gekennzeichnet,**
**dass** die Oxidation durch PbO₂, Permanganat, Cer⁴⁺-Verbindungen oder/und Peroxodisulfat erfolgt.

13. Verfahren nach einem der Ansprüche 1 bis 12,
**dadurch gekennzeichnet,**
**dass** die Reduktion räumlich oder/und zeitlich getrennt von der Oxidation erfolgt.

14. Verfahren nach einem der Ansprüche 1 bis 13,
**dadurch gekennzeichnet,**
**dass** der naszierende Wasserstoff in unmittelbarer Nachbarschaft des Metallkomplexes= erzeugt wird.

15. Verfahren nach einem der Ansprüche 1 bis 14,
**dadurch gekennzeichnet,**
**dass** der naszierende Wasserstoff elektrochemisch erzeugt wird.

16. Verfahren nach Anspruch 15,
**dadurch gekennzeichnet,**
**dass** die elektrochemische Erzeugung durch Anlegen einer Spannung von ≤ -1,0 V bezogen auf eine Ag/AgCl-Referenzelektrode erfolgt.

17. Verfahren nach einem der Ansprüche 1 bis 14,
**dadurch gekennzeichnet,**
**dass** der naszierende Wasserstoff chemisch erzeugt wird.

18. Verfahren nach Anspruch 17,
**dadurch gekennzeichnet,**
**dass** die chemische Erzeugung durch Li/Butanol/H₂SO₄, Zn-Cu/Ethanol oder Zn/HCI erfolgt.

19. Verfahren nach einem der Ansprüche 1 bis 14,
**dadurch gekennzeichnet,**
**dass** der naszierende Wasserstoff mittels Ultraschall erzeugt wird.

20. Verfahren nach Anspruch 19,
**dadurch gekennzeichnet,**
**dass** die Erzeugung mittels Ultraschall durch Abstraktion von Wasserstoff-Radikalen aus organischen Verbindungen, insbesondere Alkyl verbindungen, erfolgt.

21. Verfahren nach einem der Ansprüche 1 bis 8,
umfassend eine chemische Oxidation des Metallkomplexes und eine elektrochemische Erzeugung des naszierenden Wasserstoffs.

22. Verfahren nach einem der Ansprüche 1 bis 21,
**dadurch gekennzeichnet,**
**dass** Oxidation und Erzeugung des naszierenden Wasserstoffes in zwei getrennten Reaktionskammern erfolgt.

23. Vorrichtung zum Nachweis eines Analyten in einer Probe unter Verwendung eines lumineszierenden Metallkomplexes als Markierungsgruppe, umfassend:
(i) Mittel zum Oxidieren des Metallkomplexes,
(ii) Mittel zum Erzeugen von naszierendem Wasserstoff und
(iii) Mittel zum Nachweis von Chemilumineszenz
**dadurch gekennzeichnet,**
**dass** die Mittel (i) und (ii) zwei separate Reaktionskammern umfassen.

24. Vorrichtung nach Anspruch 23
**dadurch gekennzeichnet,**
**dass** Mittel (i) zur chemischen Oxidation des Metallkomplexes vorgesehen sind.

25. Vorrichtung nach einem der Ansprüche 23 oder 24,
**dadurch gekennzeichnet,**
**dass** Mittel (ii) zur elektrochemischen Erzeugung von naszierendem Wasserstoff vorgesehen sind.

26. Verfahren zur Erzeugung von Chemilumineszenz, umfassend das Bereitstellen eines lumineszierenden Metallkomplexes das Oxidieren des Metallkomplexes und das Reduzieren des Metallkomplexes durch naszierenden Wasserstoff, wobei eine chemilumineszenzfähige Form des Metallkomplexes entsteht, **dadurch gekennzeichnet, dass** Oxidation und Reduktion in zwei getrennten Reaktionskammern erfolgen.

27. Vorrichtung zur Erzeugung von Chemilumineszenz umfassend:
(i) Mittel zum Oxidieren eines lumineszierenden Metallkomplexes und
(ii) Mittel zum Erzeugen von naszierendem Wasserstoff
**dadurch gekennzeichnet,**
**dass** die Mittel (i) und (ii) zwei separate Reaktionskammern umfassen.

## Claims

1. Method for detecting an analyte in a sample using a luminescent metal complex as a labelling group,
**characterized in that**
the chemiluminescence of the metal complex is excited by the steps:
(i) oxidizing the metal complex and
(ii) reducing the metal complex by nascent hydrogen to form a form of the metal complex that is capable of chemiluminescing and
(iii) determining the analyte by means of the chemiluminescence.

2. Method as claimed in claim 1,
**characterized in that**
a metal complex is used as a labelling group which contains a structure of the general formula (I):
(M(L₁L₂L₃)]ₙ-Yₘ- (I)
in which
M is a divalent or trivalent metal cation selected from rare earth or transition metal cations,
L₁, L₂ and L₃ are the same or different and denote ligands containing at least two nitrogen-containing heterocycles, where L₁, L₂ and L₃ are bound to the metal cation by nitrogen atoms,
Y denotes a linker bound to one of the ligands,
m is an integer from 1 to 10 and
n is an integer from 1 to 6.

3. Method as claimed in claim 1 or 2,
**characterized in that**
a ruthenium complex is used as the metal complex.

4. Method as claimed in one of the claims 1 to 3,
**characterized in that**
the ligands of the metal complex are selected from bipyridine or phenanthroline ring systems.

5. Method as claimed in one of the claims 1 to 4,
**characterized in that**
the metal complex contains at least one hydrophilic group or/and a charge carrier.

6. Method as claimed in one of the claims 1 to 5,
**characterized in that**
the metal complex is used as a conjugate with a detection reagent for the analyte.

7. Method as claimed in one of the claims 1 to 6,
**characterized in that**
the detection is carried out as a homogeneous test.

8. Method as claimed in one of the claims 1 to 6,
**characterized in that**
the detection is carried out as a heterogeneous test.

9. Method as claimed in one of the claims 1 to 8,
**characterized in that**
the metal complex is oxidized electrochemically.

10. Method as claimed in claim 9,
**characterized in that**
the oxidation takes place by applying an anodic potential of at least + 1.2 V (relative to an Ag/AgCl reference electrode).

11. Method as claimed in one of the claims 1 to 8,
**characterized in that**
the metal complex is oxidized chemically.

12. Method as claimed in claim 11,
**characterized in that**
it is oxidized by PbO₂, permanganate, Cer⁴⁺ compounds or/and peroxodisulfate.

13. Method as claimed in one of the claims 1 to 12,
**characterized in that**
the reduction is separated spatially or/and in time from the oxidation.

14. Method as claimed in one of the claims 1 to 13,
**characterized in that**
the nascent hydrogen is generated in the direct vicinity of the metal complex.

15. Method as claimed in one of the claims 1 to 14,
**characterized in that**
the nascent hydrogen is generated electrochemically.

16. Method as claimed in claim 15,
**characterized in that**
the electrochemical generation is carried out by applying a voltage of ≤ -1.0 V relative to an Ag/AgCl reference electrode.

17. Method as claimed in one of the claims 1 to 14,
**characterized in that**
the nascent hydrogen is generated chemically.

18. Method as claimed in claim 17,
**characterized in that**
it is chemically generated by Li/butanol/H₂SO₄, Zn-Cu/ethanol or Zn/HCl.

19. Method as claimed in one of the claims 1 to 14,
**characterized in that**
the nascent hydrogen is generated by means of ultrasound.

20. Method as claimed in claim 19,
**characterized in that**
the generation by means of ultrasound takes place by abstraction of hydrogen radicals from organic compounds and in particular from alkyl compounds.

21. Method as claimed in one of the claims 1 to 8,
comprising a chemical oxidation of the metal complex and an electrochemical generation of the nascent hydrogen.

22. Method as claimed in one of the claims 1 to 21,
**characterized in that**
the oxidation and generation of nascent hydrogen take place in two separate reaction chambers.

23. Device for the detection of an analyte in a sample using a luminescent metal complex as a labelling group comprising:
(i) means for oxidizing the metal complex,
(ii) means for generating nascent hydrogen and
(iii) means for detecting chemiluminescence,
**characterized in that**
the means (i) and (ii) comprise two separate reaction chambers.

24. Device as claimed in claim 23,
**characterized in that**
the means (i) are provided for the chemical oxidation of the metal complex.

25. Device as claimed in one of the claims 23 or 24,
**characterized in that**
the means (ii) are provided for the electrochemical generation of nascent hydrogen.

26. Method for generating chemiluminescence comprising the provision of a luminescent metal complex, oxidizing the metal complex and reducing the metal complex by nascent hydrogen to form a form of the metal complex that is capable of chemiluminescing, **characterized in that** oxidation and reduction take place in two separate reaction chambers.

27. Device for generating chemiluminescence comprising:
(i) means for oxidizing a luminescent metal complex and
(ii) means for generating nascent hydrogen,
**characterized in that**
the means (i) and (ii) comprise two separate reaction chambers.

## Revendications

1. Procédé de détection d'un analyte dans un échantillon en utilisant un complexe métallique luminescent en tant que groupe de marquage,
**caractérisé en ce que**,
une chimiluminescence du complexe métallique est stimulée par les étapes
(i) d'oxydation du complexe métallique et
(ii) de réduction du complexe métallique au moyen d'hydrogène généré in situ, une forme pouvant être chimiluminescente du complexe métallique se formant et
(iii) de détermination de l'analyte par la chimiluminescence.

2. Procédé selon la revendication 1,
**caractérisé en ce que**,
on utilise comme groupe de marquage un complexe métallique contenant une structure de formule générale (I) :
[M(L₁L₂L₃)]ₙ-Yₘ-
dans laquelle
M est un cation métallique bi- ou trivalent choisi parmi des cations de terre rare ou des cations de métal de transition,
L₁, L₂, L₃ sont identiques ou différents et représentent des ligands comprenant au moins deux hétérocycles contenant de l'azote, L₁, L₂ et L₃ étant liés par l'atome d'azote au cation métallique,
Y représente le segment de liaison lié à un des ligands et
m est un nombre entier de 1 à 10 et
n est un nombre entier de 1 à 6.

3. Procédé selon la revendication 1 ou 2,
**caractérisé en ce que**,
on utilise comme complexe métallique un complexe de ruthénium.

4. Procédé selon l'une quelconque des revendications 1 à 3,
**caractérisé en ce que**,
les ligands du complexe métallique sont choisis parmi des combinaisons cycliques de bipyridine ou de phénanthroline.

5. Procédé selon l'une quelconque des revendications 1 à 4,
**caractérisé en ce que**,
le complexe métallique contient au moins un groupe hydrophile et/ou un porteur de charge.

6. Procédé selon l'une quelconque des revendications 1 à 5,
**caractérisé en ce que**,
le complexe métallique est utilisé comme conjugat avec un réactif de détection pour les analytes.

7. Procédé selon l'une quelconque des revendications 1 à 6,
**caractérisé en ce que**,
la détection est réalisée au moyen d'un test homogène.

8. Procédé selon l'une quelconque des revendications 1 à 6,
**caractérisé en ce que**,
la détection est réalisée au moyen d'un test hétérogène.

9. Procédé selon l'une quelconque des revendications 1 à 8,
**caractérisé en ce que**,
l'oxydation du complexe métallique se fait électrochimiquement.

10. Procédé selon la revendication 9,
**caractérisé en ce que**,
l'oxydation se fait par application d'un potentiel anodique d'au moins +1,2 V (ramené à une électrode de référence Ag/AgCl).

11. Procédé selon l'une quelconque des revendications 1 à 8,
**caractérisé en ce que**,
l'oxydation du complexe métallique se fait chimiquement.

12. Procédé selon la revendication 11,
**caractérisé en ce que**,
l'oxydation se fait au moyen de composés PbO₂, permanganate, Cer⁴⁺ et/ou de peroxodisulfate.

13. Procédé selon l'une quelconque des revendications 1 à 12,
**caractérisé en ce que**,
la réduction se fait séparément dans l'espace et/ou dans le temps de l'oxydation.

14. Procédé selon l'une quelconque des revendications 1 à 13,
**caractérisé en ce que**,
l'hydrogène généré in situ est fabriqué à proximité immédiate du complexe métallique.

15. Procédé selon l'une quelconque des revendications 1 à 14,
**caractérisé en ce que**,
l'hydrogène généré in situ est fabriqué électrochimiquement.

16. Procédé selon la revendication 15,
**caractérisé en ce que**,
la génération électrochimique se fait par application d'une tension de ≤ -1,0 V ramené à une électrode de référence Ag/AgCl.

17. Procédé selon l'une quelconque des revendications 1 à 14,
**caractérisé en ce que**,
l'hydrogène généré in situ est fabriqué chimiquement.

18. Procédé selon la revendication 17,
**caractérisé en ce que**,
la génération chimique se fait au moyen de Li/butanol/H₂SO₄, ZnCu/éthanol ou Zn/HCl.

19. Procédé selon l'une quelconque des revendications 1 à 14,
**caractérisé en ce que**,
l'hydrogène généré in situ est généré au moyen d'ultrasons.

20. Procédé selon la revendication 19,
**caractérisé en ce que**,
la génération par ultrasons se fait par abstraction de radicaux hydrogène de composés organiques, en particulier de composés alkyles.

21. Procédé selon l'une quelconque des revendications 1 à 8,
comprenant une oxydation chimique du complexe métallique et une génération électrochimique de l'hydrogène généré in situ.

22. Procédé selon l'une quelconque des revendications 1 à 21,
**caractérisé en ce que**,
l'oxydation et la génération de l'hydrogène généré in situ se fait dans deux chambres de réaction séparées.

23. Dispositif de détection d'un analyte dans un échantillon en utilisant un complexe métallique luminescent en tant que groupe de marquage,
comprenant :
(i) un moyen d'oxydation du complexe métallique,
(ii) un moyen pour la génération d'hydrogène généré in situ et
(iii) un moyen de détection de chimiluminescence,
**caractérisé en ce que**,
les moyens (i) et (ii) comprennent deux chambres de réactions séparées.

24. Dispositif selon la revendication 23,
**caractérisé en ce que**,
le moyen (i) est prévu pour l'oxydation chimique du complexe métallique.

25. Dispositif selon l'une quelconque des revendications 23 ou 24,
**caractérisé en ce que**,
le moyen (ii) est prévu pour la génération électrochimique d'hydrogène généré in situ.

26. Procédé de génération de chimiluminescence, comprenant la préparation d'un complexe métallique luminescent, l'oxydation du complexe métallique et la réduction du complexe métallique au moyen d'hydrogène généré in situ, une forme pouvant être chimiluminescente du complexe métallique se formant, **caractérisé en ce que** l'oxydation et la réduction se font dans deux chambres de réactions séparées.

27. Dispositif de génération de chimiluminescence, comprenant :
(i) un moyen d'oxydation d'un complexe métallique luminescent et
(ii) un moyen de génération d'hydrogène généré in situ,
**caractérisé en ce que**,
les moyens (i) et (ii) comprennent deux chambres de réaction séparées.
